Europäisches Patentamt

(19) European Patent Office

Office européen des brevets

(11) Publication number: **0 082 637**
A2

# (12) EUROPEAN PATENT APPLICATION

(21) Application number: **82306559.4**

(22) Date of filing: **09.12.82**

(51) Int. Cl.³: **C 12 P 13/24**
C 12 N 15/00, C 12 N 1/20
//C12R1/125

(30) Priority: **18.12.81 JP 204577/81**

(43) Date of publication of application:
**29.06.83 Bulletin 83/26**

(84) Designated Contracting States:
**DE FR GB**

(71) Applicant: **AJINOMOTO CO., INC.**
**5-8, Kyobashi 1-chome, Chuo-ku**
**Tokyo 104(JP)**

(72) Inventor: **Tsuchida, Takayasu**
**No. 1730-13, Kamikurata-cho Totsuka-ku**
**Yokohama-shi Kanagawa-ken(JP)**

(72) Inventor: **Kawashima, Nobuki**
**No. 2-20-8, Kannon Kawasaki-ku**
**Kawasaki-shi Kanagawa-ken(JP)**

(72) Inventor: **Nakamori, Shigeru**
**No. 2153-187, Kamariya-cho Kanazawa-ku**
**Yokohama-shi Kanagawa-ken(JP)**

(72) Inventor: **Enei, Hitoshi**
**No. 2-30-2, Ikego**
**Zushi-shi Kanagawa-ken(JP)**

(72) Inventor: **Kurahashi, Osamu**
**No. 958, Kashimada Saiwai-ku**
**Kawasaki-shi Kanagawa-ken(JP)**

(74) Representative: **Bond, Bentley George et al,**
**Haseltine Lake & Co. 28 Southampton Buildings**
**Chancery Lane**
**London WC2A 1AT(GB)**

(54) **L-histidine-producing microorganisms.**

(57) L-histidine producing microorganisms are constructed by incorporating, into a recipient strain of the genus *Bacillus*, a recombinant plasmid containing a DNA fragment controlling resistance to a histidine-antagonist, which DNA fragment is obtained from chromosomal DNA of a mutant of the genus *Bacillus* resistant to the histidine-antagonist.

EP 0 082 637 A2

-1-

## L-HISTIDINE-PRODUCING MICROORGANISMS

This invention relates to microorganisms of the genus <u>Bacillus</u> constructed by a gene splicing technique, for producing L-histidine by fermentation.

In the past, in order to render a wild microorganism strain capable of producing L-histidine from carbohydrates, it has been necessary to induce artificial mutants from the wild strain. In this regard, there are many known L-histidine producing artificial mutants.

Examples of known histidine producing microorganisms include mutants of <u>Brevibacterium</u> and <u>Corynebacterium</u> resistant to 2-thiazolealanine (US-A-3716453), a mutant of <u>Corynebacterium</u> resistant to histidine-analogue and purine-analogue (JP-B-18798/1977), mutants of <u>Serratia</u> (US-A-3902966), mutants of <u>Proteus</u> (JP-A-92588/1972), mutants of <u>Brevibacterium</u> resistant to 2-thiazolealanine and sulfa-drug (JP-B-23594/1976).

Another approach to increase the histidine productivity of microorganisms is suggested in JP-A-165798/1979. In this technique <u>Escherichia</u> <u>coli</u> strains are transformed with a recombinant plasmid DNA constructed by a gene splicing technique, to produce L-histidine producing microorganisms.

However, a need still exists to produce L-histidine by fermentation in a higher efficiency than in the known methods.

According to the present invention, there is provided an L-histidine producing microorganism constructed by incorporating, into a recipient strain of the genus Bacillus, a recombinant plasmid DNA containing a DNA fragment controlling resistance to a histidine-antagonist and obtained from chromosomal DNA of a mutant of the genus Bacillus resistant to the histidine-antagonist.

The present invention also provides an L-histidine producing microorganism constructed by incorporating a first recombinant plasmid into a first recipient strain of the genus Bacillus, said first recombinant plasmid containing a DNA fragment controlling resistance to a histidine-antagonist and obtained from a transformant of the genus Bacillus, which transformant has been constructed by incorporating a second hybrid plasmid into a second recipient strain of the genus Bacillus, said second recombinant plasmid containing a DNA fragment controlling resistance to a histidine-antagonist and obtained from a mutant of the genus Bacillus resistant to a histidine-antagonist; said second recipient strain of the genus Bacillus being an L-histidine requiring strain and said first recipient strain of the genus Bacillus being resistant to a histidine-antagonist.

The histidine-antagonists used in the present invention inhibit the growth of Bacillus, but the inhibition is suppressed partly or completely when L-histidine coexists in the medium. Examples of the histidine-antagonists are 1,2,4-triazolealanine, 2-thiazolealanine, α-methylhistidine and 3-amino-1,2, 4-triazolealanine.

Although any mutant of the genus Bacillus resistant to histidine-antagonist can be used as the DNA-donor for the chromosomal DNA fragment controlling resistance to the histidine-antagonist, mutants having a higher resistance to the histidine-antagonist are preferred.

In many cases, better results can be obtained when mutant ving a higher productivity of L-histidine are used as the DNA-donor. The mutant resistant to a histidine-antagonist can be obtained in a conventional manner such as by exposing the parent strain to 250 µg/ml of N-methyl-N'-nitro-N-nitrosoguanidine, and isolating the strain capable of growing in a medium containing an amount of the histidine-antagonist inhibitive to the growth of the parent strain.

The extraction of chromosomal DNA can be carried out in a convention manner, such as described in Bacteriol, 89, 1065 (1965).

As the vector DNA, plasmid DNAs which propagate in hosts of Bacillus are used. Typical vector DNAs are pCT 127, pC 194, pC 221, pC 223 and pUB 112 (Proc. Natl. Acad. Sci. U.S.A., 74, 1680-1682 (1977)), pUB 110 (J. Bacteriol., 134, 318-329 (1978)), and pTP 4 and pTP 5 (Microbiol. Letters, 5, 55-59 (1978)), all of which are derived from plasmids of Staphylococcus, and pLS 15 and pLS 28 (J. Bacteriol., 131, 699-701 (1977)), pLS 13 (J. Bacteriol., 129, 1487-1494 (1977)), and pPL and pPL 2 (J. Bacteriol. 124, 484 (1975), all of which are derived from plasmids of Bacillus.

The chromosomal DNA is digested with a restriction endonuclease by a known method (see, for example, Biochem. Biophys. Acta, 383, 457, (1975)). Various kinds of restriction endonucleases can be used, the degree of digestion being controlled by changing the reaction time.

The vector DNA is also cleaved with a restriction endonuclease. Suitable restriction endonuclease for each vector DNA are disclosed in the literature shown in the parenthesis above.

Recombination of the DNA to prepare the recombinant plasmid can be carried out by a ligation reaction with a ligase, or by incorporating, with terminal transferase, deoxyadenylic acid and thymidylic acid (or deoxyguanylic acid and deoxycytidylic acid) into the chromosomal DNA fragment and the cleaved vector DNA and by subjecting the modified chromosomal DNA fragment and the cleaved DNA to an annealing reaction.

The recombinant DNA thus obtained can be incorporated into the DNA-redipient, for example (a) by treating cells of the DNA-recipient with calcium chloride to increase the permeability (for example as is reported in respect of E. coli K-12 by M. Mandel and A. Higa in J. Mol. Biol., 53, 159 (1970)); or (b) by using for the incorporation, cells of the DNA-recipient at a specific stage of growth when the cells become capable of incorporating plasmids (competent cell) (for example as is reported in respect of Bacillus subtilis by C. H. Duncan, G. A. Wilson and F. E. Young in Gene 1, 153 (1977)); or (c) by forming a protoplast or spheroplast of the DNA-recipient which easily incorporates plasmid DNA (for example as is known in respect of Bacillus subtilis, actinomycetes and yeast from S. Chang and S.N. Cohen, Molec. Gen. Genet, 168, 111 (1979), M. J. Bibb, J. M. Ward and O. A. Hopwood, Nature, 274, 398 (1978), and A. Hinnen, J. B. Hicks and G. R. Fink, Proc. Natl. Acad. Sci., USA, 75, 1929 (1978)).

The recipients for the recombinant DNA are microorganisms of the genus Bacillus. It is convenient to use, as the DNA recipient, microorganisms sensitive to a histidine-analogue and requiring histidine for growth for the selection of a transformant containing a recombinant DNA containing a chromosomal DNA fragment controlling resistance to a histidine-antagonist. When a recombinant plasmid DNA containing a chromosomal DNA fragment controlling resistance to a histidine-antagonist is used for the transformation, after selection of the recombinant plasmid DNA using a host sensitive to a histidine-antagonist and requiring L-histidine for growth, microorganisms resistant to a histidine-antagonist and having no requirement of L-histidine for growth can be used as the DNA recipient.

The desired transformants are those which become resistant to the histidine-antagonist and are capable of producing L-histidine when microorganisms sensitive to the histidine-antagonist and requiring L-histidine for growth are used as the recipients. When microorganisms resistant to the histidine-antagonist and having no requirement of L-histidine for growth are used, the desired transformants are those which have the characteristics possessed by the vector DNA as the maker for selection.

The methods of cultivation of the L-histidine producing transformants thus obtained are conventional, and are similar to the methods for the cultivation of known L-histidine producing microorganisms. The aqueous culture medium employed may be a conventional one containing a carbon source, a nitrogen source, inorganic ions and, when required, minor organic nutrients such as vitamins and amino acids.

As to the carbon source, carbohydrates (such as glucose, sucrose, lactose and fructose) and raw materials containing such saccharides (such as starch hydrolysate, molasses and fruit juice) may be used. Gaseous ammonia, aqueous ammonia, ammonium salts and other nitrogen containing materials can be used as the nitrogen source.

Cultivation of the microorganism is effected under aerobic conditions, the pH and the temperature of the aqueous culture medium preferably being adjusted to a suitable level previously determined, and may be continued until production of L-histidine ceases.

The invention will now be illustrated by the following Example.

Example

(1) Extraction of chromosomal DNA

Bacillus subtilis AJ 11733 (FERM-BP 217), which requires L-arginine and L-leucine and is resistant to 1,2,4-triazolealanine, was cultured in 1 litre of "Bact Penassay Broth" (Difco) at 30°C for 2 hours with shaking, and cells in the exponential growth phase were harvested. Chromosomal DNA was extracted from the cells by a conventional phenol-method (J. Bacteriol., 89, 1065)(1965)), whereby 4.0 mg of purified DNA were obtained.

(2) Insertion of the chromosomal DNA fragment into a vector

As the vector, pUB 110 possessing genetic information relating to kanamycin-resistance and neomycin-resistance was used. 10 µg of the chromosomal DNA obtained in step (1) and 5 µg of the vector DNA were digested separately with the endonuclease Eco RI at 37°C for 1 hour, and thereafter the two reaction mixture were heated at 65°C for 10 minutes and mixed. The mixed solution was subjected to a ligation reaction by a $T_4$ phage DNA-ligase in the presence of ATP and dithreitol at 10°C for 24 hours.

(3) Genetic transformation with the plasmid containing the histidine producing gene

Bacillus subtilis AJ 11732 (FERM-BP 224), which requires L-arginine, L-leucine and L-histidine, was cultured in "Penassay Broth" (Difco) at 30°C overnight with shaking, thereafter cultured at 37°C for 4 hours with shaking in Medium-I (containing 0.5 g/dl of glucose, 0.2 g/dl of $(NH_4)_2SO_4$, 0.6 g/dl of $KH_2PO_4$, 1.4 g/dl of $K_2HPO_4$, 0.02 g/dl of $MgSO_4.7H_2O$, 0.1 g/dl of sodium citrate, 0.2 g/dl of yeast extract, 10 mg/dl of L-histidine, 25 mg/dl of L-arginine and 5 mg/dl of L-leucine), and further cultured, after the cultivation in Medium-I, at 37°C for 1.5 hours with shaking in Medium-II (containing 0.5 g/dl of glucose, 0.2 g/dl of $(NH_4)_2SO_4$, 0.6 g/dl of $KH_2PO_4$, 1.4 g/dl of $K_2HPO_4$, 0.12 g/dl of $MgSO_4.7H_2O$, 0.1 g/dl of sodium citrate, 0.02 g/dl of yeast extract, 5 mg/dl of L-arginine and 0.5 mg/dl of L-leucine). Thus, competent cells having the ability of plasmid uptake were obtained (C. Anagnostopoulos, J. Spizizen, J. Bacteriol., 81, 741, (1961)).

To a suspension of the competent cells, there was added the recombinant plasmid containing the histidine producing gene obtained in step (2), and incubation at 37°C for 2 hours with shaking was carried out to complete the transformation reaction.

The cell-suspension was transferred onto a minimum medium prepared by adding 5 µg/ml of kanamycin, 10 mg/dl of L-leucine, 10 mg/dl of L-arginine, 50 mg/dl of 1,2,4-triazolealanine and 2 g/dl of agar to a basal minimum medium having a pH of 7.2 and containing 0.6 g/dl of $KH_2PO_4$, 1.4 g/dl of $K_2HPO_4$, 0.2 g/dl of $(NH_4)_2SO_4$, 0.1 g/dl of sodium citrate, 0.02 g/dl of $MgSO_4 \cdot 7H_2O$, and 0.5 g/dl of glucose. After 3 days of cultivation at 37°C, three colonies appeared on the agar medium.

Among the three transformants, Bacillus subtilis AJ 11734 (FERM-BP 218), which had the highest productivity of L-histidine, was selected. The DNAs in Bacillus subtilis AJ 11734 were extracted from it by C.I. Kado's phenol method (J. Bac., 145, 3, 1365 (1981)). The plasmid DNA and chromosomal DNA were separated by agarose-gel electrophoresis, and the plasmid DNA obtained was purified by dialysis.

The purified plasmid was incorporated into Bacillus subtilis AJ 11733, which produces L-histidine, in the manner described in step (3), and as the desired transformant, kanamycin and 1,2,4-triazolealanine-resistant Bacillus subtilis AJ 11735 (FERM-BP 219) was obtained.

(4) Histidine production by the new histidine producers

The L-histidine productivity of Bacillus subtilis AJ 11733, Bacillus subtilis AJ 11734 and Bacillus subtilis AJ 11735 was tested as follows. Batches each of 20 ml, of a culture medium having a pH of 7.0 and containing, per decilitre, 8 g of glucose, 1 g of $NH_4Cl$, 0.2 g of KCl, 0.1 g of $KH_2PO_4$, 0.04 g of $MgSO_4.7H_2O$, 0.4 g of "casamino acid" (Difco), 1 mg of $FeSO_4.4H_2O$, 1 mg of $MnSO_4.4H_2O$, 20 mg of L-arginine, 20 mg of L-leucine and 4 g of $CaCO_3$, were placed in 500 ml shaking flasks. In addition, 5 μg/ml of kanamycin were added to the medium for Bacillus subtilis AJ 11734 and Bacillus subtilis AJ 11735.

Cultivation was carried out at 30°C for 96 hours with shaking. The amounts of L-histidine in the supernatant of the resulting culture media were determined by microbiological assay, and are shown in the Table.

Table

| Microorganism tested | L-Histidine accumulated (mg/dl) |
|---|---|
| Bacillus subtilis AJ 11733 | 110 |
| Bacillus subtilis AJ 11734 | 150 |
| Bacillus subtilis AJ 11735 | 230 |

(6) Recovery of Bacillus subtilis AJ 11732

Bacillus subtilis AJ 11732 can be easily obtained by removing the plasmid from Bacillus subtilis AJ 11734 in a conventional manner such as follows. Bacillus subtilis AJ 11734 was cultured with shaking in 4 ml of CMG-2 medium having a pH of 7.0 and containing 0.5 g/dl of glucose, 1 g/dl of yeast extract, 1 g/dl of peptone and 0.5 g/dl of NaCl, placed in a 20 ml test tube. The temperature was adjusted at 30°C from initiation to 12 hours, and at 41°C from 12 hours to 36 hours. Cells in the resulting culture broth were collected, suspended in sterilized water, and spread on a CMG-2-agar-plate. The plate was then incubated at 30°C for one day, and was replicated onto a second CMG-2-agar-plate containing 10 μg/ml of kanamycin. The second CMG-2-agar-plate was incubated at 30°C for 1 day. The strain which could not grow on the second CMG-2-agar-plate was separated

as <u>Bacillus</u> <u>subtilis</u> AJ 11732.

<u>Rule 28(1) Information</u>

<u>Bacillus</u> <u>subtilis</u> AJ 11732, <u>Bacillus</u> <u>subtilis</u> AJ 11733, <u>Bacillus</u> <u>subtilis</u> AJ 11734 and <u>Bacillus</u> <u>subtilis</u> AJ 11735 have the taxonomic characteristics as disclosed in Proc. Sec. Int. Congr. Microbiol. (London, 1936) 245 (1937) and Nomencl. Comm. Intern. Soc. Microbiol., 28, (1937), and in addition have characteristics as disclosed hereinabove.

<u>Bacillus</u> <u>subtilis</u> AJ 11733, <u>Bacillus</u> <u>subtilis</u> AJ 11734 and <u>Bacillus</u> <u>subtilis</u> AJ 11735 were deposited at the Fermentation Research Institute, Japan, on the 11th December 1981 under the accession numbers FERM-BP 217, FERM-BP 218 and FERM-BP 219, respectively, which deposits were converted to deposits under the Budapest Treaty on the 22nd November 1982. <u>Bacillus</u> <u>subtilis</u> AJ 11732 was deposited at the Fermentation Research Institute, Japan, on the 11th December 1981 under the accession number FERM-BP 217, which deposit was converted to a deposit under the Budapest Treaty on the 27th November 1982.

CLAIMS

1. An L-histidine producing microorganism constructed by incorporating, into a recipient strain of the genus <u>Bacillus</u>, a recombinant plasmid DNA containing a DNA fragment controlling resistance to a histidine-antagonist and obtained from chromosomal DNA of a mutant of the genus <u>Bacillus</u> resistant to the histidine-antagonist.

2. A microorganism as claimed in claim 1, wherein said mutant and/or said recipient strain are of the species <u>Bacillus</u> <u>subtilis</u>.

3. A microorganism as claimed in claim 1 or 2, wherein said histidine-antagonist is 1,2,4-triazolealanine.

4. A microorganism as claimed in any of claims 1 to 3, wherein said recipient strain requires L-histidine for growth.

5. A microorganism as claimed in any of claims 1 to 4, wherein said recipient strain is resistant to a histidine-antagonist.

6. An L-histidine producing microorganism constructed by incorporating a first recombinant plasmid into a first recipient strain of the genus <u>Bacillus</u>, said first recombinant plasmid containing a DNA fragment controlling resistance to a histidine-antagonist and obtained from a transformant of the genus <u>Bacillus</u>, which transformant has been constructed by incorporating a second hybrid plasmid into a second recipient strain of the genus <u>Bacillus</u>, said second recombinant plasmid containing a DNA fragment controlling resistance to a histidine-antagonist and obtained from a mutant of the genus <u>Bacillus</u> resistant to a histidine-antagonist; said second recipient strain of the genus <u>Bacillus</u> being an L-histidine requiring strain and said first recipient strain of the genus <u>Bacillus</u> being resistant to a histidine-antagonist.

7. A microorganism as claimed in claim 6, wherein

**0082637**

said mutant and/or said first recipient strain and/ or said second recipient strain are of the species Bacillus subtilis.

8. Bacillus subtilis FERM-BP 218.

9. Bacillus subtilis FERM-BP 219.

10. A method of producing L-histidine, which comprises aerobically culturing an L-histidine producing microorganism as claimed in any of claims 1 to 9 in an aqueous culture medium.